# EUROPEAN PATENT SPECIFICATION

(11) **EP 1 032 651 B1**
(45) Date of publication and mention of the grant of the patent: **16.08.2006**
(21) Application number: 98955534.7
(22) Date of filing: 22.10.1998
(51) Int. Cl.: C12N 5/10, A61K 35/12

(54) **INDUCIBLE CELLULAR IMMUNITY THROUGH ACTIVATION OF Th1 AND SUPPRESSION OF Th2 RESPONSES BY MACROPHAGES**
INDUZIERTE ZELLULÄRE IMMUNITITÄT DURCH AKTIVIERUNG DER TH1 UND UNTERDRÜCKUNG DER TH2 ANTWORT DURCH MACROPHHAGEN
IMMUNITE CELLULAIRE INDUCTIBLE PAR L'ACTIVATION DE REACTIONS DEPENDANTES DE TH1 ET LA SUPPRESSION DE REACTIONS DEPENDANTES DE TH2 AU MOYEN DE MACROPHAGES

(30) Priority: 23.10.1997 EP 97203286
(43) Date of publication of application: 06.09.2000
(73) Proprietor: Vlaams Interuniversitair Instituut voor Biotechnologie vzw., 9052 Zwijnaarde (BE)
(72) Inventor: DESMEDT, Marjory, B-9000 Gent (BE); FIERS, Walter, B-9070 Destelbergen (BE); GROOTEN, Johan, B-9920 Lovendegem (BE)
(86) International application number: PCT/EP1998/006986
(87) International publication number: WO 1999/021968

(56) References cited:
- WO-A-96/05288
- SOLDERA S. ET AL.,: "Selective down-regulation of the Th2 immune responses following treatment with antigen-coupled splenocytes" EUR. J. IMMUNOL., vol. 27, - April 1997 pages 848-854, XP002060388
- CONSTANT S. L. & BOTTOMLY K.: "Induction of Th1 and Th2 CD4+ T cell responses: the alternative approach" ANNU. REV. IMMUNOL., vol. 15, - 1997 pages 297-332, XP002060443
- FITZGERALD T.J.: "The Th1/Th2-like switch in syphilitic infection: is is detrimental" INFECTION AND IMMUNITY, vol. 60, no. 9, - September 1992 pages 3475-3479, XP002060389
- ROITT I. M.: "Encyclopedia of immunology" , ACADEMIC PRESS , 1992 XP002060392 see page 114 - page 115
- SCOTT P.: "IFN-gamma modulates the early development of Th1 and Th2 responses in a murine model of cutaneous leishmaniasis" J. IMMUNOL., vol. 147, no. 9, - 1 November 1991 pages 3149-3155, XP002060390
- NABORS G. S. ET AL.,: "Switch from a type 2 to a type 1 T helper cell response and cure of established Leishmania major infection in mice is induced by combined therapy with interleukin 12 and pentostam" PROC. NATL. ACAD. SCI. USA, XP002060391
- TRANNOY E. ET AL.,: "Monoclonal c-myc transformed macrophage cell lines" J. IMMUNOL., vol. 151, no. 6, - 15 September 1993 pages 3042-3056, XP002060444
- DESMEDT M. ET AL.,: "Macrophages induce cellular immunity by activating Th1 cell responses and suppressing Th2 cell responses" J. IMMUNOLOGY, vol. 160, no. 11, - 1 June 1998 pages 5300-5308, XP002092609

## Description

The invention concerns the use of macrophages in the function of antigen presenting cells (APC) for the manufacture of a medicament to redirect the balance of the CD4⁺ T helper 1 (Th1 ) / CD4⁺ T helper 2 (Th2) cell subsets during an immune response. More particularly, the present invention is directed to pharmaceutical formulations comprising macrophages or a cell population containing macrophages, optionally together with a suitable excipient, for use in methods for suppressing the Th2 dependent humoral immune response and/or to stimulate the Th1 dependent, cellular immune response.

### Background of the invention

Upon T cell receptor (TCR) - ligation, naïve CD4⁺ T cells, referred to as Th0 cells, differentiate into distinct subsets characterised by their functions and cytokine production profiles (1). Thus Th1 lymphocytes, characterised by the production of IL-2, IFN-γ and TNF-β, contribute to cellular immunity whereas Th2 lymphocytes, mainly involved in humoral immunity, produce IL-4, IL-5 and IL-10. Numerous examples of the consequences on disease outcome of skewed Th1 to Th2 ratios have been reported. Polarised Th2 responses have been implicated in pathological situations, such as *Leishmania major* (2, 3), TBC (4) human leprosy (5), and mycotic infections (6). The contribution of Th1 cells relative to Th2 cells to the developing autoimmune response determines for a large part whether or not this response leads to a clinical disease (7-9). In allergic asthma, a predominant Th2 response has been noted (10). Also the chronic autoimmune graft-versus-host disease, which develops after the administration of mismatched lymphoid cells, can be prevented by switching a Th2 to a Th1 response through administration of IFN-γ at the time of cellular transfer (11). Moreover, Roussel et al. (12) describe that the inefficiency of the immune response against a human glioma is caused by the presence of activated tumor-infiltrating lymphocytes, characterised by a predominant type 2 lymphokine production. These cytokines do not promote a tumoricidal immune response and therefore do not counteract the growth of the tumor.
As a consequence, parameters that control Th1/Th2 development may play a crucial role in the susceptibility or resistance to a particular disease, especially so because individual naïve T helper cells appear capable of differentiating into either T-cell subset. Besides parameters like MHC haplotype (13), dose and nature of the antigen (14, 15), and the route of antigen administration (16), the availability of IFN-γ and IL-12 as opposed to IL-4 is decisive for the maturation into Th1 and Th2, respectively, both in vitro and in vivo (reviewed in 17). Initial sources of IL-12 and IFN-γ are mainly cells that are part of the innate immune system, namely macrophages, dendritic cells and natural killer cells. The cells responsible for the initial production of IL-4 are less well defined and apparently include the naïve T-cells themselves, induced by IL-6 (17). Because it is likely that most in vivo responses do not take place in a milieu with sufficient levels of cytokines, professional APCs may steer Th0 maturation towards Th1 or Th2 by providing, besides the ligands for the TCR and costimulatory receptors, also the necessary cytokines. Accordingly, dendritic cells seem to induce preferentially the development of Th1 cells (19). However, other authors describe that dendritic cells regulate both cellular and humoral immune responses (20). B-cells on the other hand, seem to support preferentially the induction and expansion of Th2 T-cells (21). Some organisms, such as nematodes, secrete cytokine like substances that cause a Th2 up-regulating activity, resulting in a down-regulation of Th1 activity (22). Soldera and coworkers (23) recently demonstrated that a selective down-regulation of Th2 dependent IgG1 production can be obtained by treatment of mice with antigen-coupled splenocytes. These splenocytes, however, are a complex mixture of different cell types and it is not clear whether the effect is due to only one of the subpopulations of the splenocytes or to a cooperative effect of two or more subpopulations.

### Detailed description of the invention

In this invention, it is demonstrated that syngeneic macrophages that have been pulsed ex vivo with exogenous protein induced a primary immune response, characterised by a predominant activation of Th1 reactivity. The macrophages used in these experiments as an example were derived from a clonal, immortalized population that both functionally as well as phenotypically expressed features characteristic of mature macrophages. The macrophages were Mf4/4 cells; they expressed the surface molecules BM-8, F4/80, Mac-1, Mac-2 and CD14 that have been described for mature macrophages, exerted receptor-mediated phagocytosis and produced IL-1, IL-6, IL-12 and TNF in response to LPS but not IFN-γ. Moreover, the Mf4/4 cells expressed increased levels of MHC class II antigens after treatment with IFN-γ and concomitantly acquired the capacity to present exogenous antigen to CD4⁺ T-cells. These results demonstrate that, despite their transformed state, the Mf4/4 cells retained their macrophage-specific constitutive and inducible functions.
Mf4/4 cells were cocultured for a short time with antigen and subsequently injected into syngeneic mice. It was inferred that these cells presented in vivo antigen-derived antigenic epitopes and that this resulted in a primary T cell response, because restimulation of splenocytes with antigen gave a proliferative response while this was absent in naïve splenocytes. This proliferative response was observed only when injected macrophages were first treated with IFN-γ and cocultured with antigen, and when the spleen APC, presenting the antigen in the secondary response, possessed the same MHC haplotype as the injected macrophages. The latter finding indicates that the induced immunity was a consequence of antigen presentation by the injected macrophages rather then by endogenous APC that acquired the antigenic peptides through exchange of MHC-class II bound peptides or through capture of membrane-bound antigen.
Analysis of the cytokine secretion pattern by the enzyme-linked immunospot technique (ELISPOT) revealed that the ratio of IFN-γ to IL-4 producing antigen-reactive cells was about 10 fold higher in mice immunised with antigen-pulsed macrophages in comparison to mice immunised with soluble antigen or antigen emulsified in adjuvant. Clearly, this shift towards a Th1 cytokine profile had functional implications. Thus, the anti-antigen antibody response that was induced by a subsequent immunisation with soluble antigen contained, besides the T cell independent IgM antibodies, a remarkably high titer of T cell dependent IgG antibodies. The latter belonged to the Th1 dependent IgG2 isotype, while the IgG1 subtype, characteristic of humoral immunity, was absent. This particular IgG isotype profile was in strong contrast to the development of, besides IgG2a and IgG2b, IgG1 anti-antigen antibodies in mice immunised with soluble antigen or antigen emulsified in adjuvant. In order to verify whether other macrophage clones share with Mf4/4 this capacity for an exclusive activation of cellular immunity, the same experiment was performed with 3 additional macrophage clones, derived from an independent immortalisation experiment and exhibiting similar activities in vitro as Mf4/4. All three clones proved to be as potent as Mf4/4 in priming mice for IgG2a and IgG2b antibody responses and, like Mf4/4, did not prime for IgG1. This result indicates that the effect is not a clone-specific phenomenon. It can be concluded that mature macrophages which have been induced by IFN-γ to present exogenous antigen, are potent inducers of Th1 reactivity and cellular immunity.
Of particular interest is the additional observation that administration of antigen-loaded macrophages in between injections of free antigen, resulted in the complete suppression of Th2-dependent IgG1 production.
As a consequence the observed exclusive induction of Th1-derived cellular immunity, as well as the observed suppression of Th2-derived humoral immunity by IFN-γ-treated macrophages, defines these cells as "Th1 APC".

Although the results described have been obtained with immortalised macrophage clones, the full retention of mature macrophage-specific features by the cells supports the assumption that, provided they are appropriately induced by IFN-γ, macrophages exert Th1-APC activity in the organism. As a result, they skew Th0 cell responses towards Th1 maturation, suppress Th2 responses and, as a result thereof, promote cellular immune responses.
As already mentioned above, mice injected with antigen-pulsed macrophages generated a strong IgG response upon a boost with soluble antigen. This result was quite unexpected due to the lack of B-cell reactivity during priming, apparent from the absence of anti-antigen antibodies. Whereas the latter observation can be explained by the inaccessibility of the Mf4/4-bound antigen to the B-cell receptor, the strength of the secondary, in fact primary B-cell response indicates that the level of available T-cell help rather than a previous encounter with antigen of the reactive B-cells is critical for IgG production.
A first aspect of the present invention thus relates to the use of antigen presenting macrophages for the manufacture of a medicament to redirect the Th1/Th2 balance towards an exclusive Th1 response. Influencing or redirecting this balance can be used in a number of applications. It can be used in a number of diseases where a suppression of the humoral Th2 cell reactivity is required, without affecting or even promoting the Th1 subset. Such diseases are e.g. Leishmaniasis, TBC and some mycotic infections as Candidiasis. Another application of this invention is the manufacture of a medicament for treatment of Th2-dependent autoimmune diseases as well as allergic diseases, which are also Th2-dependent, such as allergic asthma and rhinitis.
A second aspect of the present invention relates to manufacture of a medicament for the generation of an IgG response in absence of B-cell reactivity during priming. This aspect of the invention is useful if vaccination is required but injection of the antigen is potentially harmful, for example because of the toxicity of the antigenic protein.
A further aspect of the present invention relates to the manufacture of a medicament for modulation of the population of the immunoglobulin isotypes, especially the downregulation of Th2 cell dependent immunoglobulin isotypes, such as IgG1 or IgE and/or the upregulation of Th1 cell dependent antibody responses such as IgG2a and IgG2b.
Part of the invention is also the use of antigen presenting macrophages, or a cell population comprising antigen presenting macrophages for the manufacture of a pharmaceutical composition to manipulate the CD4⁺ Th1 / Th2 balance. Furthermore to the invention belongs a pharmaceutical composition comprising antigen presenting macrophages or a cell population comprising antigen presenting macrophages, optionally together with a suitable excipient known to a person skilled in the art.
In addition, the invention also concerns a pharmaceutical composition comprising the antigen in an appropriate formulation such that it promotes, preferably, a selective uptake and presentation of said antigen by macrophages of the recipient itself whereafter the CD4⁺ Th1 / Th2 balance can be influenced in either way.
On this basis a cure of- for example- allergic asthma and other allergic diseases requires enforcing macrophages of the patient, pre-activated or not by administration of interferon-γ, to present the allergen to CD4⁺ T cells. By converting the self-amplifying pathological process of Th2 activation / Th1 suppression to its opposite namely Th1 activation / Th2 suppression, a long term cure of the allergic disease is expected from this treatment.
Enforcing the macrophages of the patient to present the allergen, is achieved by coupling said allergen to molecules with affinity for macrophage-specific receptors that mediate endocytosis of the receptor-bound molecules. Endocytosis of the allergen enables the macrophage to process the allergen and, subsequently, to present the allergen to allergen-specific CD4⁺ T lymphocytes.

The present invention will be further described with reference to the examples below, which are only intended by way of explanation and do not imply any limitation whatsoever to the scope of the invention.

### EXAMPLES

### MATERIALS AND METHODS

### 1) Mice

Female C57BI/6 (H-2^{b}) and BALB/c (H-2^{d}) mice were purchased from Broekman (Eindhoven, Netherlands). All mice were 9-14 weeks of age at the time of the experiments.

### 2) Immortalisation of macrophages

Immortalisation of macrophages was performed as described (24). Briefly, primary cultures from spleens were plated at a density of 10⁶ cells/ml. The cells were grown in RPMI-1640 medium (Life Technologies, Paisley, UK), supplemented with 10 % foetal bovine serum (FBS; Life Science International, Zellik, Belgium), L-glutamine (2mM), penicillin (100U/ml), streptomycin (100µg/ml), sodiumpyruvate (1mM) (Life Technologies) and 2-ME (5x10⁻⁵M; BDH, Poole, UK). One day after seeding, cells were infected with VN11 retrovirus released by N11 producer cells. Briefly, 0.5 ml of N11 fresh supernatant from a 24 h subconfluent culture was filtered on 0.22µm filters (Costar Co., Cambridge, USA), diluted 1/1 with complete medium containing 10 µg/ml polybrene (Sigma Chemical Co., St. Louis, USA), and added to the primary cultures for 1 h at 37°C. Once established, the cells were cloned by limiting dilution.

### 3) Immunofluorescence

The phenotype of the cell clones was determined by indirect immunofluorescence on living cells using a set of monoclonal antibodies. Primary antibodies were R-phycoerythrin conjugated anti-Mac-1 (CD11 b), anti-CD14, anti-CD18, anti-FcγRII (CD32), anti-CD71 (Pharmingen, San Diego, USA), anti-Mac-2 (Cedarlane Laboratories, Hornby, Canada), anti-BM-8, anti-ER-MP58 (BMA Biochemicals, Augst, Switzerland), anti-F4/80, anti-CD40, biotinylated anti-I-A^{b} (Serotec, Oxford, UK), anti-B7-1 (CD80) and anti-B7-2 (CD86) (gift from K. Thielemans). FITC-conjugated goat anti-rat IgG (FITC-GAR) (Life Technologies) was used as secondary antibody except for detection of anti-B7-1 and biotinylated antibodies, where FITC-conjugated goat anti-hamster (Sera-lab, Sussex, UK) and streptavidin (Serotec), respectively, were used. Where mentioned, cells were pre-treated for 16 h with 400 U/ml of recombinant murine IFN-γ (IFN-γ) or 10 µg/ml lipopolysaccharide (LPS; Difco Laboratories, Detroit, USA). Analysis was performed using a FACSCalibur cytofluorimeter (Becton Dickinson, Erembodegem, Belgium).

### 4) Phagocytosis of erythrocytes

Sheep red blood cells (SRBC), were opsonised by mixing one volume of fresh SRBC (10% suspension, Sigma) with an equal volume of appropriately diluted anti-SRBC antiserum (Sigma) for 30 minutes at room temperature. Subsequently, erythrocytes were washed, plated on monolayers of macrophages at a ratio of 50:1 and incubated at 37°C for 1 h in RPMI medium supplemented with 10% FBS. After removal of free SRBC by medium exchanges and lysis by osmotic shock, the cells were fixed, stained with May-Grünwald Giemsa and examined under the microscope for ingestion of SRBC.

### 5) Phagocytosis of fluorescent microspheres

Fluorescent microspheres were purchased from Duke Scientific Corporation (USA) and opsonised with goat anti-mouse Ig antibodies. Serial dilution of the microspheres was made in phosphate buffered saline (PBS), sonicated for 30 sec and added to 10⁵ cells. After incubation for 1h at 37°C, unbound microspheres were separated from cells by passage through a percoll gradient. Cells containing ingested microspheres were detected and quantified by flow cytometry. In order to distinguish membrane-binding from internalisation, a negative control was included consisting of samples incubated at 4°C. This temperature prohibits active processes such as phagocytosis but not membrane binding.

### 6) Cytokine determination

Macrophages were harvested from adherent cultures grown in tissue culture flasks using an enzyme-free cell dissociation buffer (Life Technologies) and plated at a cell density of 3x10⁴/200µl in flat bottom 96 microwell plates (Falcon, Becton Dickinson, Franklin Lakes, USA). After 24 h, cells were stimulated with 20 µg/ml LPS or 200 U/ml IFN-γ for 2 more days and the culture fluid was collected.

The levels of IL-1 and IL-6 were quantified as described previously by the proliferation of cell line D10(N4)M (25) and 7TD1 (26) respectively. TNF production was assayed using the WEHI-164 cytotoxicity assay (27). IL-12 levels were determined by sandwich EIA (Biosource International, Camarillo, USA). The assay detects the heterodimeric IL-12 protein as well as the p40 subunit.

### 7) T-HA proliferation assay

The haemagglutinin (HA)-specific and H-2^{b} restricted CD4⁺ T cell clone T-HA was developed in our laboratory by initial immunisation of C57BI/6 mice with 10µg HA and 0.5 mouse dose Ribi adjuvant containing 25µg monophosporyl lipid A (MPL) and 25µg of trehalose dimycolate (TDM) (RIBI Immuochem Research, Hamilton, USA) and a second immunisation with 3µg HA 3 weeks later. 5 days after this boost immunisation, lymph nodes were isolated and 3.10⁷ cells were stimulated in vitro with 0.5µg/ml HA in 25cm² culture flasks. On day 4, 10U/ml murine IL-2 (from PMA-stimulated EL4.IL-2 cells) was added to the cultures. After 2 additional bi-weekly restimulations with 0.5µg/ml HA and APC, a pool of optimally HA-reactive T-Iymphocytes was obtained. T-HA cells were maintained long-term in vitro by biweekly restimulation in 25cm² culture flasks with 200ng/ml HA and 7.10⁷ syngeneic spleen cells from C57BI/6 mice (3000 rad g-irradiated). On day 2, 30 IU/ml of recombinant human IL-2 was added, after which T cells were further cultured and expanded by medium renewal and IL-2 addition every 4 days. The cytokine secretion profile of antigen-stimulated T-HA cells was typical for Th1 T-cells namely production of IFN-γ and lack of IL-4.
The antigen HA is the major surface glycoprotein of influenza virus and was prepared by digestion of purified X47 virus with bromelain (28), purified by ion exchange on a DEAE column (Pharmacia, Uppsala, Sweden), and revealed as a single band on a silver stained SDS-PAGE. The HA preparations were free of endotoxin.
Mf4/4 macrophages were seeded in 96-well flat bottom microtiter plates at a density of 2.10⁴ cells/well. After 24 h, the indicated concentrations of HA were added in combination with IFN-γ (400 U/ml) or LPS (10µg/ml) and the cells were further cultured overnight. The following day, macrophages were treated with mitomycin C (50µg/ml) (Duchefa, Haarlem, the Netherlands) for 90min at 37°C, thoroughly washed and 1.10⁴ T-HA T-cells were added to each well together with indomethacin (40µM; Sigma) and catalase (1248U/ml; Sigma). After 72 h, 0.5µCi/well of [³H]thymidine (Amersham Life Science, Amersham, UK) was added for an additional 16 h of culture. Cells were harvested on glass fibre filters and the incorporated radioactivity was measured by liquid scintillation in a TopCount (Packard Instruments Co., Meriden, CT). Results are means of triplicate cultures.

### 8) Immunisation and proliferative response of immunised spleen cells

Mice were immunised against HA by intraperitoneal (i.p.) injection of 2.5µg HA, dissolved in 200µl PBS or emulsified in 0.5 mouse dose Ribi adjuvant. Alternatively, mice were injected with Mf4/4 macrophages that present HA-derived peptides. The latter were derived from Mf4/4 cultures, maintained for 48 h in FBS-free medium supplemented with 10mg/l insulin, 5.5mg/l transferrin, 6.7µg/l selenium (ITS; Gibco), and to which IFN-γ (400 U/ml) and HA (1µg/ml) were added for the last 24 h. The so treated cells were harvested, washed extensively with PBS in order to remove free proteins, and immediately injected. Repeated injections were given at biweekly intervals. Mice immunised with Ribi adjuvant received an emulsion of HA and 25µg MPL (RIBI) as adjuvant in the boost injection. Unless otherwise mentioned, experimental groups were made up of 2 animals. The proliferative response to HA of the immunised spleen cells was assayed in 96-well flat bottom microtiter plates as described above. Briefly, the immunised mice were sacrificed, their spleens removed and the splenocytes seeded at 2.10⁵ cells/well. Unless otherwise mentioned, 1µg/ml HA was added to the cultures. After 72 h, cell proliferation was measured by [³H]thymidine incorporation. Immunisation experiments were repeated at least once.

### 9) Cytokine ELISPOT assay

The frequency of antigen-induced IFN-γ or IL-4 producing T cells was determined using the enzyme-linked immunospot (ELISPOT) technique according to the protocol proposed by Pharmingen. Groups of mice were immunised i.p. with HA-pulsed macrophages, 2.5 µg HA in PBS, 2.5 mg HA emulsified in 0.5 mouse dose Ribi adjuvant or PBS ad placebo followed, 2 weeks later, by a second injection. 14 days after immunisation, 5.10⁷ splenocytes were stimulated ex vivo with 1µg/ml HA in 5 ml complete medium for 24 h. Viable cells were recovered from the cultures by passage through a Histopaque-1077 (Sigma Aldrich Co., Irvine, UK) density gradient, washed, seeded in nitro-cellulose bottomed 96-well Millititer HA plates (Millipore, Molsheim, France) at a density of in 4x10⁵ cells/well, and cultured for an additional 24 h. The anti-IFN-γ and anti-IL-4 capturing and biotinylated detection mAbs were purchased from Pharmingen. Spots were visualised using Avidin D-peroxidase and 3-amino-9-ethylcarbazole substrate (Sigma), and counted under a microscope. The frequency of cytokine-secreting cells was derived from the increment of number of spots detected with immunised versus placebo-treated splenocytes. No spots were seen in non-stimulated cultures where no HA was added.

### 10) Determination of anti-HA antibody titer and isotype by indirect EIA

Blood samples were taken and sera prepared 14 days after the last immunisation. The sera were serially diluted in Maxisorp 96 well plates (Nunc, Roskilde, Denmark) previously coated with HA by overnight incubation at 4°C with a 0.5µg/ml stock solution of the antigen. Bound antibody was detected with goat anti-mouse isotype-specific antibodies (anti-IgG, anti-IgG1, anti-IgG2a, anti-IgG2b, anti-IgM; Sigma) using alkaline phosphatase-conjugated rabbit anti-goat IgG as detecting antibody (Sigma). Serum samples were collected from 2 mice per group and analysed individually.

For further clarification of the invention examples are herewith provided in more detail.

### EXAMPLE 1

### Phenotypic and functional characterisation of immortalised macrophage clones

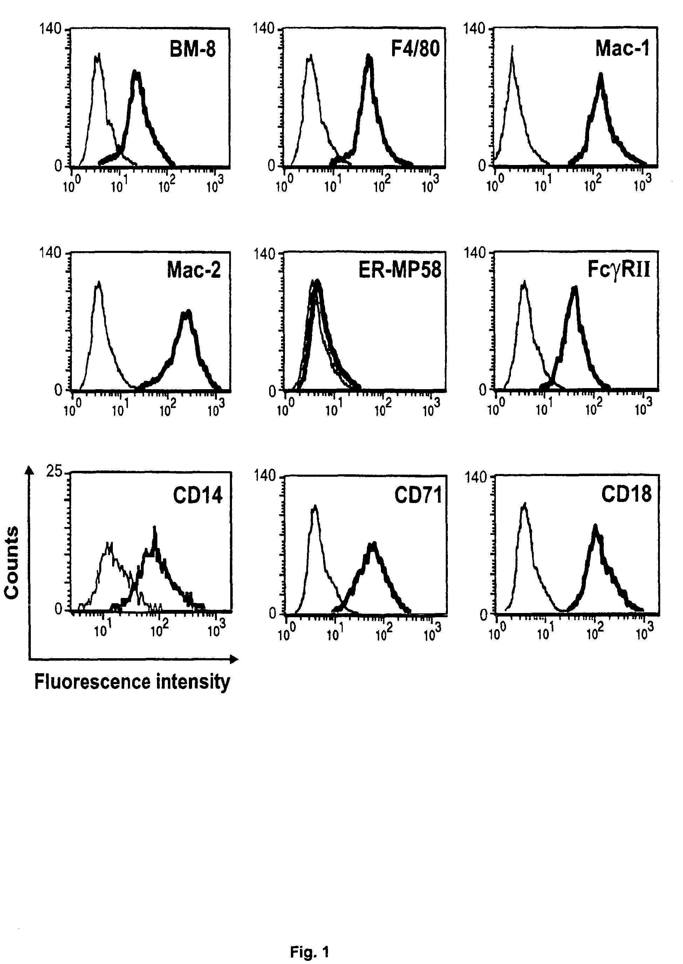

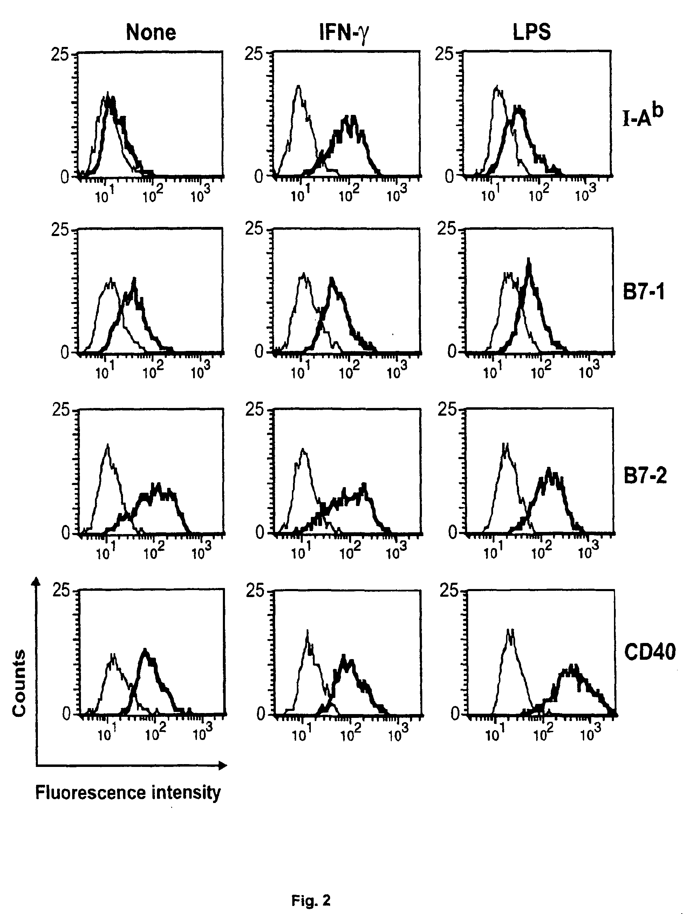

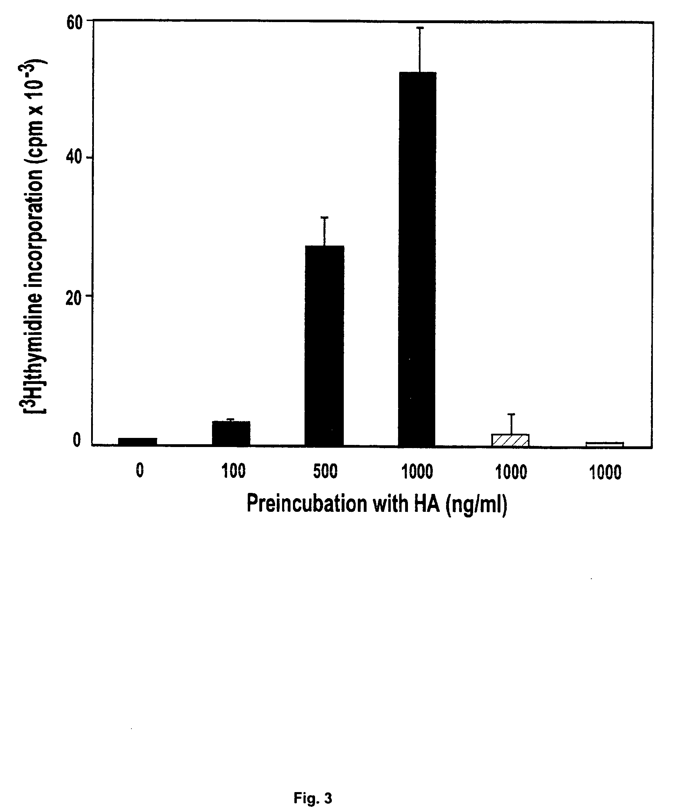

In order to determine whether macrophages were able to prime T cells in vivo, macrophages, isolated from the spleen of C57BI/6 mice, were immortalised, providing the advantage over freshly isolated cells of an unlimited source of a functionally and phenotypic homogeneous cell population. Immortalisation was performed as described before by infection of spleen cell suspensions with VN11 retroviruses (24). This resulted in the establishment of various cell lines exhibiting macrophage features. From these, clone Mf4/4 is a good representative and was used throughout further experiments. Phenotypic analysis of Mf4/4 revealed the presence of BM-8, a macrophage marker, as well as of F4/80, Mac-1 (CD11b) and Mac-2, which are only expressed by mature macrophages (Fig.1). The latter characteristic was confirmed by the absence of the immature macrophage marker ER-MP58. In addition, the cells expressed high levels of FcγRII (CD32) and CD14, both absent on dendritic cells (29), the transferrin receptor CD71, and the adhesion molecule CD18.
Functionally, Mf4/4 adhered strongly to plastic surfaces, a feature characteristic for macrophages, and exerted activities that are assigned to macrophages namely SER- and FcyR-mediated phagocytosis of sheep red blood cells (30) and opsonised fluorescent microspheres, respectively, as well as secretion of IL-1, IL-6, IL-12 and TNF-α upon treatment with LPS (Table I). These cytokines were not detectable in the culture fluid of cells stimulated with IFN-γ.
Finally, the expression levels of the ligands necessary for T cell activation were assessed. Besides TCR-ligation through MHC class II (I-A)-associated antigenic peptides, appropriate T cell activation requires interaction with costimulatory ligands, mainly B7-1 (CD80) and/or B7-2 (CD86) (31). Also the CD40-CD40L interaction, expressed by APCs and T-cells, respectively, plays a central role in antigen presentation and APC activation (32). Examination of the expression levels of these markers on the Mf4/4 cells revealed the constitutive expression of B7-1, B7-2 and CD40 (Fig. 2). Treatment with IFN-γ readily induced the expression of I-A, whereas the levels of the constitutively expressed B7-1 and B7-2 costimulatory ligands remained nearly constant. Stimulation with LPS resulted in a weak induction of I-A but a strong, five-fold increase of CD40 expression. In order to verify whether this induction of an APC⁺ phenotype (I-A⁺, B7-1⁺, B7-2⁺, CD40⁺) by LPS but especially by IFN-γ had functional implications, the capacity of the macrophages to activate the HA-specific Th1 lymphocyte population T-HA was assessed. Figure 3 shows the proliferative response of T-HA cells triggered by mitomycin-C treated Mf4/4 macrophages, previously pulsed with increasing amounts of HA and pre-treated or not with IFN-γ or LPS. Clearly, the induction of an APC⁺ phenotype by IFN-γ but not by LPS was accompanied by the acquisition of APC-activity. The absence of proliferation when HA was replaced by hen egg white lysozyme (HEL) demonstrates that the observed response was antigen specific.
The above results demonstrate that the Mf4/4 cells are phenotypically mature macrophages and exert macrophage-specific activities among which the presentation of exogenous antigen to Th1 lymphocytes. Moreover, the pivotal role of IFN-γ in regulating this feature is underlined by increasing the expression level of I-A^{b} MHC molecules, thereby converting the macrophage into a dedicated APC.

### EXAMPLE 2

### Injection of Mf4/4 macrophages, ex vivo loaded with HA, generates a primary, anti-HA T-cell response

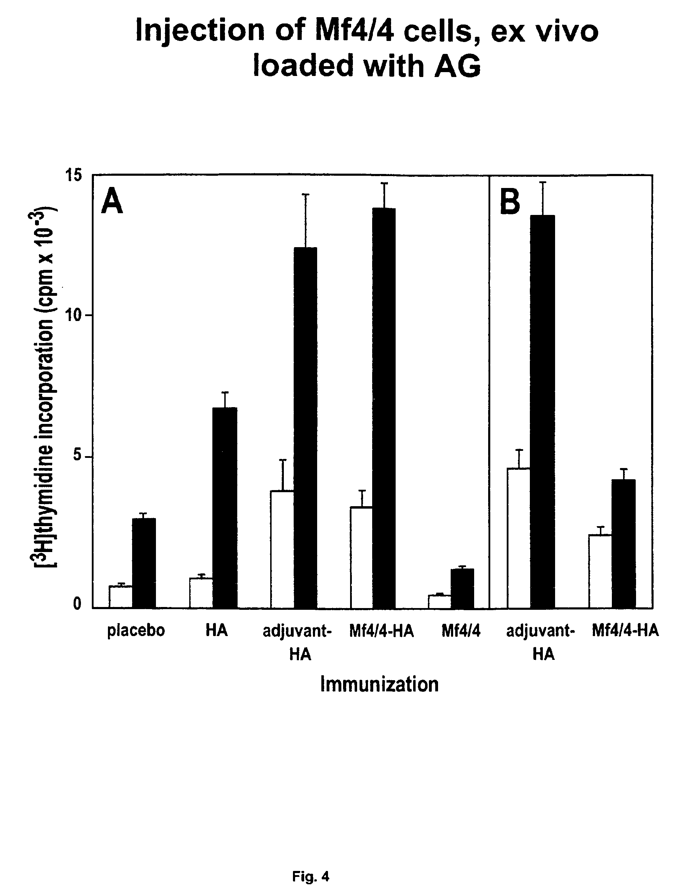

In order to assess whether mature macrophages, induced to exert APC-activity by in vitro pre-treatment with IFN-γ in the presence of an optimal concentration of HA (1 mg/ml), also exerted this activity in the animal, 1.5x10⁶ activated and loaded Mf4/4 cells were injected intraperitoneally (i.p.) into C57BI/6 mice. In order to reduce the level of irrelevant proteins and hereby obtain a maximal presentation of relevant, HA-derived peptides, the FBS present in the culture medium was replaced by a mixture of insulin, transferrin and selenium (ITS) during coculture with antigen. 14 days after injection, the mice were killed and their spleen cells assayed for occurrence of a secondary, anti-HA T-cell response (Fig. 4, panel A). Splenocytes from mice injected with HA-presenting macrophages elicited a pronounced anti-HA proliferative response. This response was significantly stronger than the one observed with spleen cells from mice injected with soluble, intact HA and was comparable to the response of mice injected with HA emulsified in adjuvant. Unlike the HA-presenting macrophages, injection of control macrophages did not prime the spleen cells for a secondary, anti-HA proliferative response, demonstrating the dependence of the priming on HA-derived peptides. However, this does not totally exclude the possibility that the peptides were presented to the host T cells by endogenous APC, having acquired the antigenic peptides on their MHC molecules through passive exchange or following uptake of debris from dead Mf4/4 cells. Accordingly, it was determined whether in MHC mismatched mice the secondary response is restricted by the H-2 haplotype of the injected macrophages viz. the recipient. As shown in figure 4, panel B, injection of HA-pulsed Mf4/4 cells (H-2^{b}) into H-2^{d} BALB/c mice did not induce a secondary, H-2^{d} restricted proliferative response, triggered by BALB/c spleen APC. Nevertheless, both strains developed comparable in vitro responses after priming with free HA, emulsified in adjuvant. Therefore, it was concluded that the absence of a secondary anti-HA response in BALB/c splenocytes, immunised with Mf4/4 macrophages can be attributed to the H-2 mismatch between the APC involved in the primary (H-2^{b}) and secondary (H-2^{d}) response, hence excluding non-specific antigen reprocessing by the host. Thus, adoptively transferred macrophages, loaded ex vivo with antigen present the derived, antigenic peptides to T cells of the recipient and hereby prime the animal against the antigen.

### EXAMPLE 3

### Macrophages activate the Th1-subset in vivo

Upon activation by antigen, naive CD4⁺ T cells differentiate into either Th1 and/or Th2 cells. In order to identify the differentiation pathway that is predominantly activated following immunisation with macrophages, the numbers of cells that released Th1 versus Th2 cytokines were analysed. In order to raise sufficiently the numbers of HA-reactive T-cells, mice were injected twice with either HA-loaded Mf4/4 cells, free HA, HA emulsified in adjuvant or PBS. Two weeks after the second injection, splenocytes were restimulated in vitro for 24 h with HA, presented by primary APC from the spleen. Living cells were recovered by passage through Histopaque and frequencies of HA-reactive Th1 and Th2 cells were assessed on the basis of the numbers of IFN-γ and IL-4 secreting cells, respectively, using ELISPOT assays. The results shown in Table II reveal that mice injected twice with antigen-pulsed macrophages developed high numbers of IFN-γ producing Th1 cells comparable to the numbers obtained with splenocytes from mice immunised with HA emulsified in adjuvant and significantly higher than the numbers observed after immunisation with free antigen. However, as concerns the Th2 cytokine IL-4, a remarkable difference was observed : mice vaccinated with HA-pulsed macrophages generated few IL-4 specific spots compared to animals that received HA emulsified in adjuvant. This differential response is clearly reflected in the ratio of IFN-γ to IL-4 producing cells where mice challenged with HA-pulsed macrophages exhibited a 10-fold increase relative to mice immunised with either free or adjuvant emulsified HA. Thus, macrophages shift the T cell response towards differentiation into Th1 cells, whereas free antigen induces a mixed Th1 and Th2 response, independent of the presence or not of adjuvant and of the overall strength of the T-cell response.

### EXAMPLE 4

### The predominant activation of Th1 cells generates cellular immunity

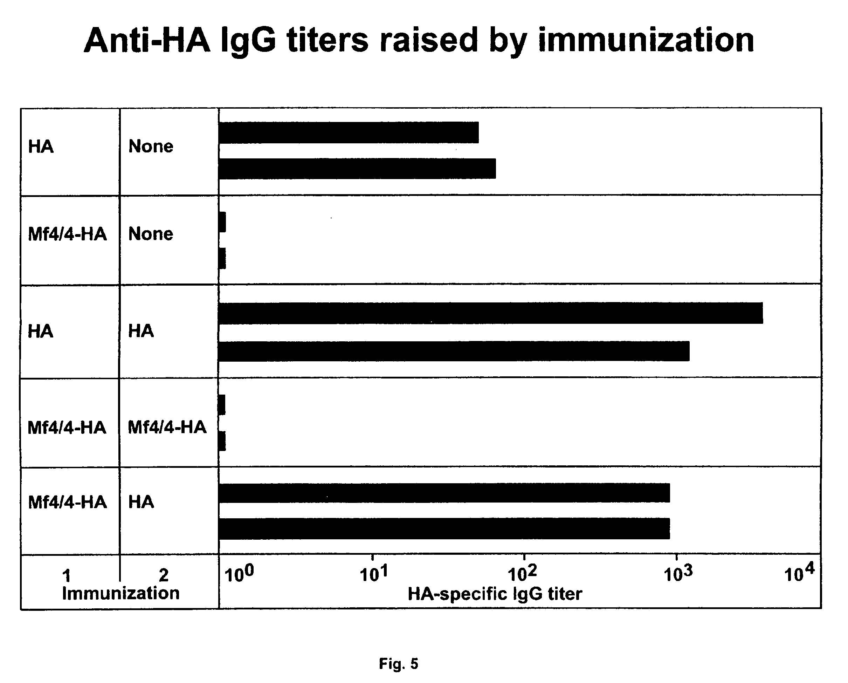

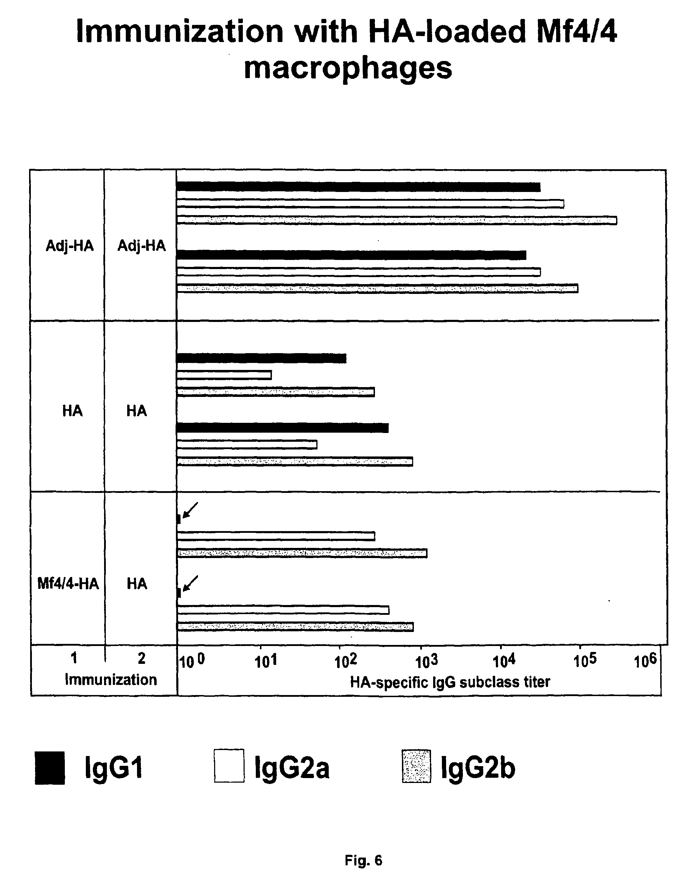

Th1 and Th2 lymphocytes differentially affect the shift from the T cell independent IgM isotype towards IgG isotypes in activated B lymphocytes, supporting a switch towards IgG2a/b and IgG1, respectively (31, 32). Accordingly, analysis of the isotype profile of anti-HA antibodies provides information regarding the preferential activation of Th1 cells upon immunisation with antigen-loaded macrophages. Therefore, mice were immunised with free HA or HA-loaded macrophages and, 14 days later, blood samples were taken and serum from individual mice was prepared and assayed. In contrast to HA-immunised mice, neither anti-HA IgM nor IgG antibodies (Fig. 5) could be detected in sera from mice immunised once or twice with HA-pulsed macrophages. However, a second injection with free HA instead of HA-loaded Mf4/4 cells induced high IgG titers, comparable to those raised by two subsequent injections of free antigen and tenfold stronger than the titers raised by a single HA injection (Fig. 5). Apparently, the absence of circulating antigen in the case of immunisation with HA-pulsed macrophages prevented antibody production although the Th cells generated promoted IgG production provided the B cells were challenged with circulating antigen.
Next, the IgG subclass titers were determined on serum samples from mice primed with free antigen or HA-pulsed macrophages followed by a boost injection with free antigen (Fig. 6). These data reveal a remarkable difference in the distribution of the anti-HA IgG subtypes : mice primed with free HA, whether or not emulsified in adjuvant, developed IgG1 as well as IgG2a and IgG2b antibodies, independent of differences in the relative strength of the response. Opposed to this, mice primed with loaded macrophages developed IgG2a and IgG2b isotypes but no IgG1. This remarkable IgG isotype composition points towards a nearly exclusive activation of Th1 cells, in agreement with the results obtained in vitro by ELISPOT.

### EXAMPLE 5

### Macrophages suppress the humoral branch of an existing immune response

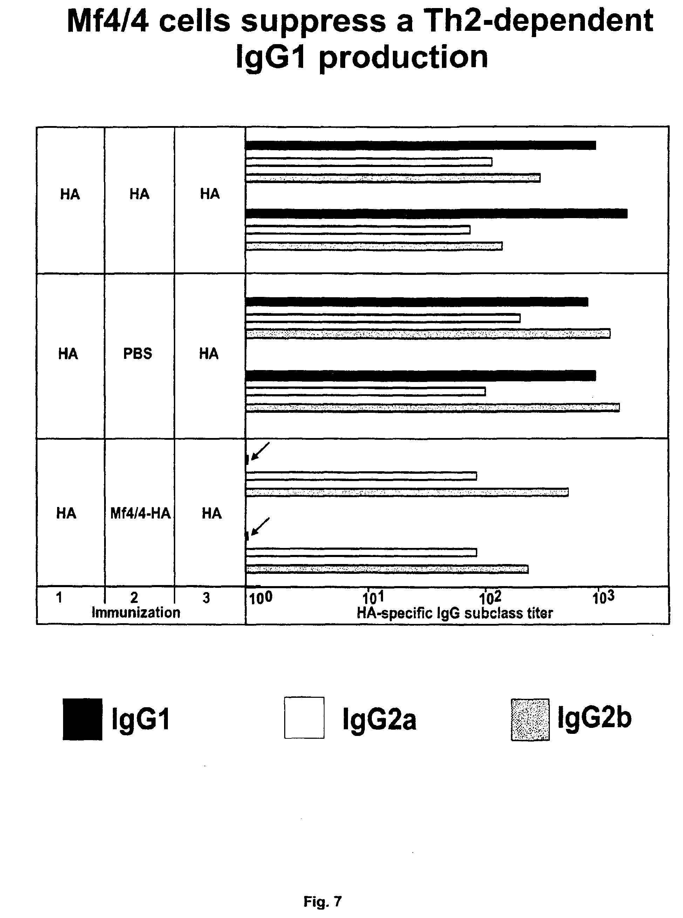

As free HA raised Th1- as well as Th2-dependent IgG responses, the impact of a subsequent immunisation with loaded macrophages was analysed on the bias of the immune response towards either IgG2 or IgG1 isotypes, respectively. To this end, mice were injected with free HA followed 2 weeks later by injection of either PBS as control, free HA, or HA-loaded macrophages. Finally, all mice were rechallenged with free HA, and the IgG isotype profile of the generated anti-HA antibodies was analysed by EIA (Fig. 7). As expected, mice which received 3 injections with free HA or where the second injection was replaced by a placebo (PBS) injection, generated comparable titers of IgG1, IgG2a and IgG2b anti-HA antibodies. However, substitution of the second injection by HA-pulsed macrophages completely abolished the IgG1 response while the levels of IgG2a and IgG2b remained unaffected. This pronounced and selective inhibition in vivo of a single isotype, is unique and indicates that, provided antigen presentation occurs exclusively through macrophages, the humoral branch of the immune response is completely and irreversibly modified. Furthermore, this result demonstrates the feasibility of redirecting a mixed immune response towards a predominant cellular response by administering antigen as peptides, presented by macrophages.

### REFERENCES

1) Mosmann, T. R., and R. C. Coffman. 1989. Th1 and Th2 cells : different patterns of lymphokine secretion lead to different functional properties. *Annu. Rev. Immunol. 7:145.*
2) Heinzel, F. P., M. D. Sadick, S. S. Mutha, and R. M. Locksley. 1991. Production of IFN-g, IL-2, IL-4 and IL-10 by CD4⁺ lymphocytes in vivo during healing and progressive murine leishmaniasis. *Proc. Natl. Acad. Sci. USA 88:7011*.
3) Nabors, G. S., L. C. C. Afonso, J. P. Farrell, and P. Scott. 1995. Switch form a type 2 to a type 1 T helper cell response and cure of established *Leishmania major* infection in mice is induced by combination therapy with interleukin 12 and pentostam. *Proc. Natl. Acad. Sci. USA 92:3142.*
4) de Jong, R., A.A. Janson, W.R. Faber, B. Naafs and T.H. Ottenhoff. 1997. IL-2 and IL-12 act in synergy to overcome antigen-specific T cell unresponsiveness in mycobacterial disease. *J. Immunol. 159:786*.
5) Yamamura, M., K. Uyemura, and R. J. Deans. 1991. Defining protective responses to pathogens: cytokine profiles in leprosy lesions. *Science 254:277.*
6) Murphy, J. W., B. A. Wu-Hsieh, L. M. Singer-Vermes, A. Ferrante, S. Moser, M. Ruso, S. A. Vaz, E. Burger, V. L. Calich, and I. C. Kowanko. 1994. Cytokines in the host response to mycotic agents. *J. Med. Vet. Mycol. 32 suppl 1:203*.
7) Racke, M. K., A. Bonomo, D. E. Scott, B. Cannella, A. Levine, C. S. Raine, E. M. Shevach, and M. Rocken. 1994. Cytokine-induced immune deviation as a therapy for inflammatory autoimmune disease. *J. Exp. Med. 180:1961.*
8) Racke, M. K., D. Burnett, S. H. Pak, P. S. Albert, B. Cannella, C. S. Raine, D. E. McFarlin, and D. E. Scott. 1995. Retinoid treatment of experimental allergic encephalomyelitis. IL-4 production correlates with improved disease course. *J. Immunol. 154:450*.
9) Leonard, J. P., K. E. Waldburger, and S. J. Goldman. 1995. Prevention of experimental autoimmune encephalomyelitis by antibodies against interleukin 12. *J. Exp. Med. 181:381.*
10) Vogel, G. 1997. New clues to asthma therapies. Science *276:1643.*
11) Donckier, V., D. Abramowicz, C. Bruyns, S. Florquin, M. L. Vanderhaeghen, Z. Amraoui, C. Dubois, P. Vandenabeele, and M. Goldman. 1994. IFN-gamma prevents Th2 cell-mediated pathology after neonatal injection of semiallogenic spleen cells in mice. J. *Immunol. 153:2361.*
12) Roussel, E., M. C. Gingras, E. A. Grimm, J. M. Bruner, and R. P. Moser. 1996. Predominance of a type 2 intratumoural immune response in fresh tumour-infiltrating lymphocytes from human gliomas. *Clin. Exp. Immunol. 105:344.*
13) Caruso, C., G. Candore, M. A. Modica, C. T. Bonanno, G. Sireci, F. Dieli, and A. Salerno. 1996. Major histocompatibility complex regulation of cytokine production. *J. Interferon and cytokine research 16:983.*
14) DeKruyff, R. H., Y. Fang, and D. T. Umetsu. 1992. IL-4 synthesis by in vivo primed keyhole limpet hemocyanine-specific CD4⁺ T-cells. Immune influence of antigen concentration and APC cell type. *J. Immunol. 149:3468.*
15) Yang, X., R. S. Gieni, T. R. Mosmann, and K. T. Hayglass. 1993. Chemically modified antigen preferentially elicits induction of Th1-like cytokine synthesis patterns in vivo. *J.exp*. *Med. 178:349.*
16) Williams, M. E., P. Caspar, I. Oswald, H. K. Sharma, O. Pankewycz, A. Sher, and S. L. James. 1995. Vaccination routes that fail to elicit protective immunity against Schistosoma mansoni induce the production of TGF-beta, which down-regulates macrophage antiparasitic activity. *J. Immunol. 154(9):4693.*
17) Constant, S. L., and K. Bottomly. 1997. Induction of Th1 and Th2 CD4⁺ T cell responses: the alternative approaches. *Annu. Rev. Immunol. 15:297*.
18) Rincon, M., J. Anguita, T. Nakamura, E. Fikrig, and R. A. Flavell. 1997. IL-6 directs the differentiation of IL-4 producing CD4⁺ T cells. J. *Exp. Med. 185:461.*
19) Macatonia, S. E., N. A. Hosken, M. Litton, P. Vieira, C. S. Hsieh, J. A. Culpepper, M. Wysocka, G. Trinchieri, K. M. Murphy, and A. O'Garra. 1995. Dendritic cells produce IL-12 and direct the development of Th1 cells from naïve CD4⁺ T-cells. *J. Immunol. 154(10):5071*.
20) Ronchese, F., B. Hausmann, and G. Le Gros. 1994. Interferon gamma and interleukin 4 producing T cells can be primed on dendritic cells in vivo and do not require the presence of B cells. *Eur. J. Immunol. 24:1148.*
21) Gajewski, T. F., M. Pinnas, T. Wong, and F. W. Fitch. 1991. Murine Th1 and Th2 clones proliferate optimally in response to distinct antigen-presenting cell populations. *J. Immunol. 146:1750.*
22) Lee, T.D.G. and V.C. Mcalister. 1996. A process of prolonging organ allograft survival. *PCT 96*/*29082*.
23) Soldera, S., S. J. McSorley, and N. Glaichenhaus. 1997. Selective down-regulation of Th2 immune responses following treatment with antigen-coupled splenocytes. *Eur. J. Immunol. 27:848*.
24) Righi, M., M. Sassano, P. Valsasnini, S. Shammah, and P. Ricciardi-Castagnoli. 1991. Activation of the M-CSF gene in mouse macrophages immortalized by retroviruses carrying a v-myc oncogene. *Oncogene 6:103.*
25) Helle, M., L. Boeije, and L. A. Aarden. 1988. Functional discrimination between interleukin 6 and interleukin 1. *Eur. J. Immunol. 18:1535.*
26) Van Snick, J., S. Cayphas, A. Vink, C. Uyttenhove, P. Coulie, M. R. Rubira, and R. J. Simpson. 1986. Purification and NH2-terminal amino acid sequence of a T-cell-derived lymphokine with growth factor activity for B-cell hybridomas. *Proc. Natl. Acad. Sci. USA 83:9679*.
27) Espevik, T., and J. Nissen-Meyer. 1986. A highly sensitive cell line, WEHI 164 clone 13, for measuring cytotoxic factor/tumor necrosis factor from human monocytes. *J. Immunol. Meth. 95:99*.
28) Brand, C. M., and J. J. Skehel. 1972. Crystalline antigen from the influenza virus envelop. *Nature New Biol 238:145.*
29) Steinman, R. M. 1991. The dendritic cell system and its role in immunogenicity. *Annu. Rev. Immunol. 9:271*
30) Crocker, P. R, S. Kelm, C. Dubois, B. Martin, A. S. Mc William, D. M. Shotton, J. C. Paulson, and S. Gordon. 1991. Purification and properties of sialoadhesin, a sialic acid-binding receptor of murine tissue macrophages. *EMBO J. 10(7):1661*
31) Hathcock, K. S., G. Laszlo, C. Pucillo, P. Linsley, and R. J. Hodes. 1994. Comparative analysis of B7-1 and B7-2 costimulatory ligands: expression and function. *J. Exp. Med. 180: 631.*
32) Grewal, I. S., and R. A. Flavell. 1996. A central role of CD40 ligand in the regulation of CD4⁺ T-cell responses. *Immunol. Today 17:410.*
33) Stevens, T. L., A. Bossie, V. M. Sanders, R. Fernandez-Botran, R. L. Coffman, T. R. Mosmann, and E. S. Vitetta. 1988. Regulation of antibody isotype secretion by subsets of antigen-specific helper T-cells. *Nature 334(6179):255.*
34) DeKruyff, R. H., L. V. Rizzo, and D. T. Umetsu. 1993. Induction of immunoglobulin synthesis by CD4⁺ T cell clones. *Sem. Immunol. 5:421*.

### Legends to the tables

**TABLE I.** Mf4/4 cells secrete pro-inflammatory cytokines upon stimulation with LPS but not upon IFN-γ treatment. Mf4/4 cells (10⁵ cells/ml) were stimulated with LPS (20µg/ml), IFN-γ (200 U/ml) or left unstimulated. After 48 h, the culture supernatants were harvested and their cytokine content assessed by bioassay (IL-1, IL-6, TNF-α) or EIA (IL-12).

**TABLE II.** Immunisation by injection of antigen-loaded Mf4/4 macrophages polarises cytokine secretion towards Th1 cytokines.
a) C57BI/6 mice were immunised twice with 2.5µg HA dissolved in PBS (HA), HA emulsified in adjuvant (adjuvant-HA), 1.5.10⁶ HA-loaded macrophages (Mf4/4-HA), or PBS at a 14 days interval.
b) Two weeks after the second injection, the splenocytes were restimulated in vitro for 24 h with 1 µg/ml HA and the numbers of HA-induced IFN-γ and IL-4 producing T cells per 4.10⁵ viable cells were determined by ELISPOT. Spleen cells from mice injected with PBS generated 18 and 1 spot(s) for IFN-γ and IL-4 respectively upon stimulation with HA. These numbers were subtracted from the counts shown. No spots were detected in unstimulated cultures.

**TABLE I. Mf4/4 cells secrete pro-inflammatory cytokines after stimulation with LPS, but not after IFN-γ treatment**

| | Cytokine secretion | |
|---|---|---|
| Stimulus | Cytokine | Concentration (ng/ml) |
| None | IL-1 | <0.001 |
| | IL-6 | <0.05 |
| | IL-12 | <0.002 |
| | TNF-α | <0.001 |
| LPS | IL-1 | 1 |
| | IL-6 | 30 |
| | IL-12 | 0.8 |
| | TNF-α | 40 |
| IFN-γ | IL-1 | <0.001 |
| | IL-6 | <0.05 |
| | IL-12 | <0.002 |
| | TNF-α | <0.001 |

Mf4/4 cells (10⁵ cells/ml) were stimulated with LPS (20 µg/ml), IFN-γ (200 U/ml) or left unstimulated. After 48h, the culture supernatants were harvested and their cytokine content was assessed by bioassay (IL-1, IL-6, TNF-α) or ELISA (IL-12).

**TABLE II. Immunization by injection of Ag-loaded Mf4/4 macrophages polarizes cytokine secretion to Th1 cytokines**

| Immunization^{a} | number of cytokine-secreting cells^{b} | | |
|---|---|---|---|
| | IFN-γ | IL-4 | IFN-γ/IL-4 ratio |
| HA | 12 ± 1 | 3 ± 0 | 4 |
| Adjuvant-HA | 163 ± 3 | 54 ± 5 | 3 |
| Mf4/4-HA | 171 ± 5 | 4 ± 0 | 43 |

| | | | |
|---|---|---|---|
| ^{a} C57BI/6 mice (n = 2) were immunized twice with 2.5 µg HA dissolved in PBS (HA), HA emulsified in adjuvant (adjuvant-HA), 1.5 x 10⁶ HA-loaded macrophages (Mf4/4-HA) or PBS at a 14-days interval. | | | |
| ^{b} Two weeks after the second injection, the splenocytes were restimulated in vitro for 24 h with 1 µg/ml HA, after which the numbers of HA-induced IFN-γ-and IL-4-producing T cells per 4 x 10⁵ viable cells were determined by ELISPOT. Spleen cells from mice injected with PBS generated 18 spots and 1 spot for IFN-γ and IL-4, respectively, after stimulation with HA. These numbers were subtracted from the counts shown. No spots were detected in unstimulated cultures. | | | |

### Legends to the figures

**FIGURE 1.** Mf4/4 cells express macrophage-specific differentiation antigens. Cell surface expression of the various differentiation antigens was assessed by indirect immunofluorescence (bold lines). The negative control was stained with the secondary antibody FITC-GAR alone (light lines).

**FIGURE 2.** Mf4/4 cells acquire an APC⁺ phenotype upon stimulation with IFN-y. Untreated cells and cells treated with IFN-γ (400U/ml) or LPS (10µg/ml) were analysed for surface expression of I-A^{b}, B7-1, B7-2 and CD40 (bold lines). Thin lines represent the fluorescence distribution of cells stained only with the second antibody.

**FIGURE 3.** APC activity of Mf4/4 cells assayed by the antigen-dependent proliferation of the HA-specific T-cells T-HA. Mf4/4 cells were either untreated (open bars) or treated with 400 U/ml IFN-γ (closed bars) or 10 µg/ml LPS (hatched bars) in the presence of the indicated concentrations of HA. The HA-induced proliferative response of the T-HA T cells was assayed by incorporation of [³H]thymidine, added during the last 16 h of the 90 h assay. Results are expressed as the mean cpm of triplicate cultures.

**FIGURE 4.** Injection of antigen-loaded Mf4/4 macrophages primes spleen cells for an antigen-specific and MHC-restricted secondary proliferative response. Mf4/4 cells were cultured for 48 h in serum-free medium, supplemented with ITS. During the last 24 h, IFN-γ (400U/ml) and HA (1µg/ml) were added to the culture (Mf4/4-HA). As negative control, IFN-γ - treated Mf4/4 cultures that had not received HA were used (Mf4/4). The pre-treated cells were injected i.p. into syngeneic C57BI/6 (A) or allogeneic BALB/c (B) mice. As additional controls, 2.5µg HA dissolved in PBS (HA) or emulsified in adjuvant (adjuvant-HA) were injected. Placebo treated mice received a single injection of PBS. The priming effect of these various immunisations was assessed on the basis of the secondary, anti-HA proliferative response of spleen cell cultures (closed bars). Open bars represent background proliferation of unstimulated cultures. Results are expressed as the mean cpm of triplicate cultures.

**FIGURE 5.** Anti-HA IgG titers raised by immunisation with HA or HA-loaded Mf4/4 macrophages. Mice were immunised once or twice with HA dissolved in PBS (HA) and/or HA-loaded macrophages (Mf4/4-HA). 14 days after the last immunisation, sera were prepared and the HA-specific IgG titers determined by EIA. Each bar represents the titer of an individual animal. Sera from naïve mice had no significant titer.

**FIGURE 6.** Immunisation with HA-loaded Mf4/4 macrophages primes for production of Th1-dependent IgG2a and IgG2b anti-HA antibodies. Sera from mice, immunised twice with HA emulsified in adjuvant (adj-HA) or dissolved in PBS (HA), and from mice injected with HA-pulsed macrophages (Mf4/4) followed by a second immunisation with HA in PBS, were tested for the levels of anti-HA IgG1 (closed bars), IgG2a (open bars) and IgG2b (hatched bars) using a subclass-specific EIA. The respective titers are grouped per individual animal. Arrows indicate the nearly total absence of HA-specific IgG1 in mice immunised with HA-loaded Mf4/4 cells followed by soluble HA.

**FIGURE 7.** Mf4/4 macrophages suppress an existing IgG1 response. Mice were injected with HA dissolved in PBS, PBS or HA-loaded macrophages (Mf4/4-HA) in between 2 injections with HA. 14 days after the last immunisation, sera were prepared and tested for the levels of anti-HA IgG1 (closed bars), IgG2a (open bars) and IgG2b (hatched bars) by subclass specific EIA. The titers are grouped per individual animal. Arrows indicate absence of IgG1 (cf. Legend to Fig. 6).

## Claims

1. The use of antigen presenting macrophages or a cell population comprising antigen presenting macrophages for the manufacture of a medicament that redirects the CD4⁺ Th1 / Th2 balance, wherein said antigen presenting macrophages are pulsed ex vivo with antigen.

2. The use according to claim 1 in which the macrophages are immortalised.

3. The use according to claim 1 or 2 to repress the CD4⁺ Th2 cell response and/or population.

4. The use according to claim 1 or 2 to promote the development of CD4⁺ Th1 cell response and/or cell population.

5. The use according to claim 1 or 2 to repress the production of Th2 dependent immunoglobulin isotypes.

6. The use according to claim 1 or 2 to repress the production of IgG1 or IgE.

7. The use according to claim 1 or 2 to promote the production of Th1 dependent immunoglobulin isotypes.

8. The use according to claim 1 or 2 to promote the production of IgG2.

9. The use according to any of the preceding claims to counteract the growth of a tumor.

10. The use according to claims 1-8 to treat Leishmaniasis, TBC, leprosy or a mycotic infection.

11. The use according to claims 1-8 to treat an autoimmune disease or chronic autoimmunity during host-versus-graft or graft-versus-host response.

12. The use according to claims 1-8 to treat allergic asthma or rhinitis.

13. The use according to claim 1, 2 or 3 to generate a CD4⁺Th1 cell response in absence of a B cell response.

14. Pharmaceutical composition comprising antigen presenting macrophages or a cell population comprising antigen presenting macrophages, optionally together with a suitable excipient, wherein said antigen presenting macrophages are pulsed ex vivo with antigen.

## Patentansprüche

1. Verwendung von antigenpräsentierenden Makrophagen oder einer Zellpopulation, die antigenpräsentierende Makrophagen umfasst, zur Herstellung eines Arzneimittels, dass das CD4⁺-Th1/Th2-Gleichgewicht umorientiert, wobei die antigenpräsentierenden Makrophagen ex vivo mit Antigen gepulst werden.

2. Verwendung nach Anspruch 1, wobei die Makrophagen immortalisiert werden.

3. Verwendung nach Anspruch 1 oder 2 zum Reprimieren der CD4⁺-Th2-Zellantwort und/oder -population.

4. Verwendung nach Anspruch 1 oder 2 zum Fördern der Entwicklung der CD4⁺-Th1-Zellantwort und/oder -Zellpopulation.

5. Verwendung nach Anspruch 1 oder 2 zum Reprimieren der Produktion von Th2-abhängigen Immunglobulin-Isotypen.

6. Verwendung nach Anspruch 1 oder 2 zum Reprimieren der Produktion von IgG1 oder IgE.

7. Verwendung nach Anspruch 1 oder 2 zum Fördern der Produktion von Th1-abhängigen Immunglobulin-Isotypen.

8. Verwendung nach Anspruch 1 oder 2 zum Fördern der Produktion von IgG2.

9. Verwendung nach einem der vorhergehenden Ansprüche zum Bekämpfen des Wachstums eines Tumors.

10. Verwendung nach den Ansprüchen 1 - 8 zum Behandeln von Leishmaniase, TBC, Lepra oder einer Pilzerkrankung.

11. Verwendung nach den Ansprüchen 1 - 8 zum Behandeln einer Autoimmunerkrankung oder chronischen Autoimmunität während einer Host-versus-Graft- oder Graft-versus-Host-Reaktion.

12. Verwendung nach den Ansprüchen 1 - 8 zum Behandeln von Bronchialasthma oder Rhinitis.

13. Verwendung nach Anspruch 1, 2 oder 3 zum Hervorrufen einer CD4⁺-Th1-Zellantwort bei Ausbleiben einer B-Zellantwort.

14. Pharmazeutische Zusammensetzung, die antigenpräsentierende Makrophagen oder eine Zellpopulation, die antigenpräsentierende Makrophagen umfasst, wahlweise zusammen mit einem geeigneten Träger umfasst, wobei die antigenpräsentierenden Makrophagen ex vivo mit Antigen gepulst werden.

## Revendications

1. Utilisation de macrophages présentant l'antigène ou d'une population cellulaire comprenant des macrophages présentant l'antigène pour la fabrication d'un médicament qui redirige l'équilibre de Th1/Th2 CD4⁺, dans laquelle lesdits macrophages présentant l'antigène sont pulsés ex vivo avec un antigène.

2. Utilisation selon la revendication 1, dans laquelle les macrophages sont immortalisés.

3. Utilisation selon la revendication 1 ou 2 pour réprimer la réponse et/ou population de cellules Th2 CD4⁺.

4. Utilisation selon la revendication 1 ou 2 pour promouvoir le développement de la réponse et/ou population de cellules Th1 CD4*.

5. Utilisation selon la revendication 1 ou 2 pour réprimer la production d'isotypes d'immunoglobuline dépendant de Th2.

6. Utilisation selon la revendication 1 ou 2 pour réprimer la production de IgG1 ou IgE.

7. Utilisation selon la revendication 1 ou 2 pour promouvoir la production d'isotypes d'immunoglobuline dépendant de Th1.

8. Utilisation selon la revendication 1 ou 2 pour promouvoir la production de IgG2.

9. Utilisation selon l'une quelconque des revendications précédentes pour contrer la croissance d'une tumeur.

10. Utilisation selon les revendications 1 à 8 pour traiter la leishmaniose, TBC, la lèpre ou une mycose.

11. Utilisation selon les revendications 1 à 8 pour traiter une maladie auto-immune ou une auto-immunité chronique au cours d'une réponse hôte contre greffe ou greffe contre hôte.

12. Utilisation selon les revendications 1 à 8 pour traiter l'asthme allergique ou la rhinite.

13. Utilisation selon la revendication 1, 2 ou 3 pour générer une réponse de cellules Th1 CD4⁺ en l'absence d'une réponse de lymphocytes B.

14. Composition pharmaceutique comprenant des macrophages présentant l'antigène ou une population cellulaire comprenant des macrophages présentant l'antigène, en option avec un excipient approprié, dans laquelle lesdits macrophages présentant l'antigène sont pulsés ex vivo avec un antigène.
